# EUROPEAN PATENT SPECIFICATION

(11) **EP 2 919 677 B1**
(45) Date of publication and mention of the grant of the patent: **15.04.2020**
(21) Application number: 13865553.5
(22) Date of filing: 10.12.2013
(51) Int. Cl.: A61F 2/16, A61B 17/00, A61B 90/00

(54) **CONTROL OF AUTOMATED INTRAOCULAR LENS INJECTORS**
STEUERUNG AUTOMATISIERTER INTRAOKULARLINSENINJEKTOREN
COMMANDE D'INJECTEURS DE LENTILLE INTRAOCULAIRE AUTOMATISÉS

(30) Priority: 19.12.2012 US 201261739058 P; 04.12.2013 US 201314096083
(43) Date of publication of application: 23.09.2015
(73) Proprietor: Alcon Inc., 1701 Fribourg (CH)
(72) Inventor: MCNEELA, Martin Anthony, Carlsbad, California 92011 (US); BOUKHNY, Mikhail, Laguna Niguel, California 92677 (US); LEUKANECH, Kurt D., Laguna Niguel, California 92677 (US)
(74) Representative: Hanna Moore + Curley
(86) International application number: PCT/US2013/074097
(87) International publication number: WO 2014/099487

(56) References cited:
- US-A- 4 836 201
- US-A1- 2008 091 135
- US-A1- 2011 092 886
- US-A1- 2011 172 676
- US-A1- 2011 264 102
- US-A1- 2011 264 102
- US-A1- 2011 302 694
- US-A1- 2012 022 548
- US-A1- 2012 296 264

## Description

### BACKGROUND

The present disclosure relates generally to control systems and methods for automated intraocular lens injectors for use in ophthalmic treatments.

Manual insertion of an intraocular lens (IOL) using an injector handpiece allows a user to precisely control the injection speed and positioning. One hand positions the handpiece and the other hand advances the lens by twisting a lead screw or by advancing a plunger mechanism similar to a syringe. Because of their manual nature, these insertion techniques provide tactile feedback to a surgeon, allowing him or her to identify adverse events, such as lens damage resulting from improper lens placement into a loading cartridge or faulty handpiece performance. In certain cases, lens damage or faulty performance could be reflected in a change in resistive force to advance the lens. It is therefore possible that under certain conditions the surgeon may be able to detect potential lens damage or faulty performance prior to advancing the lens into the eye.

These manual insertion techniques may permit a surgeon to become accustomed to the degree of forces necessary to properly advance the lens and to identify lens damage. However, such methods also are inconsistent between patients, may vary the forces on the lens, and may require a large learning curve before the surgeon can consistently and competently insert the IOL or determine when load forces may cause damage.

Power assisted insertion of an IOL, using a motorized injector handpiece for example, provides a more predictable and more consistent surgical result. However, the surgeon loses the tactile feedback relied upon by experienced surgeons using the manual methods to identify adverse events, such as damage to the IOL lens.

Therefore a need exists to provide a mechanism of force feedback to the surgeon such that a determination may be made to halt lens advancement in the event of resistive forces deviating from normal values.

US20110264102 for example is described as providing a system and method for performing an ocular surgical procedure is provided. The design provides an intra-ocular lens (IOL) to an eye through an incision in the eye by assessing IOL insertion conditions, translating said IOL insertion conditions into a desired force range for insertion of the IOL via the incision, controlling a power source to move the IOL to the eye, monitoring conditions, such as force encountered, while controlling the power source. The design employs feedback of the force encountered and selectively alters force applied based on force encountered.

The system and methods disclosed herein overcome one or more of the deficiencies of the prior art.

### SUMMARY

It will be appreciated that the scope of the invention is in accordance with the claims. Accordingly, there is provided a system in accordance with claim 1. Further features are provided in accordance with the dependent claims. The specification also includes arrangements outside the scope of the invention which have been provided as background and to assist in the understanding of the invention.

In one exemplary aspect, the present disclosure is directed to a system for implanting an intraocular lens in the lens capsule of an eye to treat an ocular condition. The device may comprise a housing having a primary axis extending between front and rear ends of the housing and may comprise a plunger longitudinally disposed within the housing and having first and second ends. The first end may be disposed towards the front end of the housing. An electric motor may be configured to cause longitudinal translation of the plunger along the primary axis of the housing. A cartridge mount at or near the front end of the housing may be configured to accommodate a removable insertion cartridge in alignment with the plunger so that an intraocular lens disposed in the insertion cartridge is displaced from the insertion cartridge as the plunger is translated towards the front end of the housing. The system also comprises a control circuit comprising a controller electrically communicating with the electric motor and configured to power the electric motor to translate the plunger and displace the intraocular lens. The controller is configured to detect motor current feedback in the control circuit and is configured to compare the detected motor current feedback to a stored current profile and to modify translation of the plunger when the current level deviates from the stored current profile by a pre-stored amount.

In one aspect, an audible indicator is configured to alert the user when the detected motor current feedback deviates from the stored current profile. The audible indicator may continuously generate sound at a variable pitch, the pitch varying in response to variances in the detected current feedback. The pitch may be configured to vary based on changes in the motor load based on the detected motor feedback. The pitch may be configured to vary based on deviation of the actual load based on the detected motor feedback from the stored current profile. The audible indicator generates a sound at a variable rate, the rate varying in response to variances in the detected motor current feedback. The audible indicator may be configured to provide real-time feedback regarding changes in load to the surgeon.

In one aspect, the stored current profile represents an expected load on the motor when translating the plunger and displacing the intraocular lens. In another aspect, a sensor is disposed to detect a longitudinal position of the plunger. In another aspect, the controller comprises a memory portion storing the stored current profile, the stored current profile representing an expected load on the motor, wherein the controller is configured to compare an actual load based on the detected motor current feedback to the stored expected load.

In another exemplary aspect, the present disclosure is directed to a system for implanting an intraocular lens in the lens capsule of an eye to treat an ocular condition. The system may comprise a housing having a primary axis extending between front and rear ends of the housing, and may have a plunger longitudinally disposed within the housing and having first and second ends. The first end may be disposed towards the front end of the housing. An electric motor may be configured to cause longitudinal translation of the plunger along the primary axis of the housing and a cartridge mount may be disposed at or near the front end of the housing and may be configured to accommodate a removable insertion cartridge in alignment with the plunger so that an intraocular lens disposed in the insertion cartridge is displaced from the insertion cartridge as the plunger is translated towards the front end of the housing. A control circuit may comprise a controller electrically communicating with the electric motor and configured to power the electric motor to translate the plunger and displace the intraocular lens. The controller may be configured to detect motor current feedback in the control circuit and configured to compare the detected motor current feedback to a stored current profile and to emit a sensory signal to the surgeon indicative of the detected current feedback.

In one exemplary aspect, the present disclosure is directed to a method of controlling a system for implanting an intraocular lens in the lens capsule of an eye to treat an ocular condition. The method may comprise storing a motor load profile in a memory, receiving an input signal to advance an intraocular lens disposed within an electrically-powered handheld IOL injection device having a motor configured to advance the intraocular lens, advancing the intraocular lens, detecting an actual load required to advance the lens by monitoring motor current feedback, comparing the actual load to the stored motor load profile, and modifying movement of the intraocular lens when the actual load deviates from the stored motor load profile by a pre-stored amount.

In one aspect, the method includes providing a sensory signal representing the actual load. Providing a sensory signal may include emitting an audible signal that changes in real-time to represent changes in the actual load. In one aspect, the audible indicator is a continuous tone. In one aspect, the audible indicator is an intermittent beeping sound. In one aspect, providing a sensory signal includes emitting an audible indicator that changes in real-time to represent the amount of deviation from the actual load.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

The accompanying drawings illustrate embodiments of the devices and methods disclosed herein and together with the description, serve to explain the principles of the present disclosure.
Fig. 1 is an illustration of an exemplary surgical system in accordance with one aspect of the present disclosure.
Fig. 2 is an illustration of an exemplary IOL injection apparatus, with an insertion cartridge installed.
Fig. 3 is an illustration of a distal end of the exemplary IOL injection apparatus showing the insertion cartridge in greater detail.
Fig. 4 is an illustration of a cross-sectional view of the exemplary IOL injection apparatus according to one exemplary aspect of the present disclosure.
Fig. 5 is an illustration of a cross-sectional view of the exemplary IOL injection apparatus in a retracted condition according to one exemplary aspect of the present disclosure.
Fig. 6 is an illustration of a cross-sectional view of the exemplary IOL injection apparatus in a partially extended condition according to one exemplary aspect of the present disclosure.
Fig. 7 is a schematic of an exemplary control circuit according to one exemplary aspect of the present disclosure.
Fig. 8 is a flow diagram of an exemplary method according to one exemplary aspect of the present disclosure.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For simplicity, in some instances the same reference numbers are used throughout the drawings to refer to the same or like parts.

The systems and methods described herein permit a surgeon to insert an IOL into an eye of patient with a predictable and consistent surgical result, while providing feedback to the surgeon regarding the loads applied on the IOL by the IOL injection device to identify adverse conditions. In the embodiments disclosed herein, the feedback is the result of monitoring the motor current being sent to the IOL injection device motor. In some embodiments, an audible aspect, such as, for example, a continuous tone with a variable pitch provides an indicator to the surgeon of changes in the motor current. By providing the surgeon with real-time audible feedback, the surgeon may make informed decisions about the process. In addition, some aspects of the systems and methods disclosed herein provide an automatic override that halts advancement of the IOL when the current falls outside an acceptable current range.

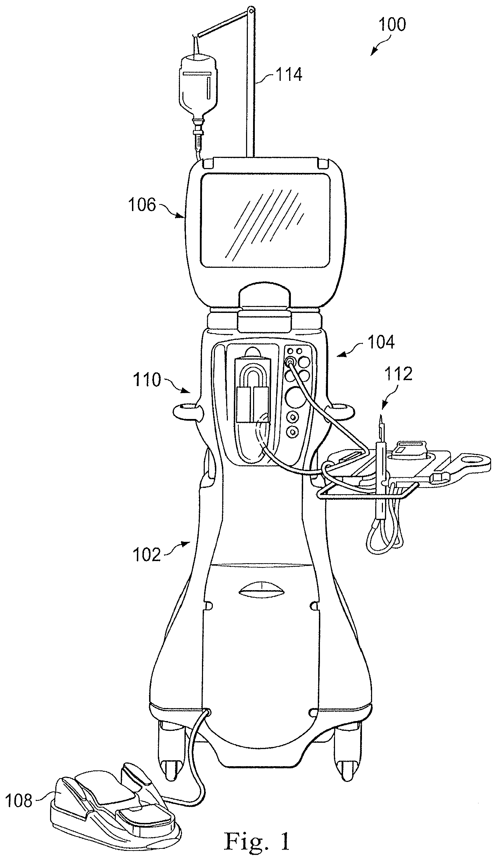

Fig. 1 illustrates a surgical console, generally designated 100, designed to treat an ocular condition, according to an exemplary embodiment. In one aspect, the surgical console 100 is particularly configured and arranged to enable a surgeon to implant an IOL into the eye of the patient. The console 100 includes a base housing 102 with a processing unit 104 and an associated display screen 106 showing data relating to system operation and performance during an IOL surgical procedure. The console 100 also includes a number of cooperating subsystems that are used together to perform the surgical procedures. For example, the subsystems include, among others, a footpedal 108, a fluidics subsystem 110, an injector subsystem including a motorized handheld intraocular lens (IOL) injection device 112, and an intravenous (IV) pole subsystem 114 including a motorized IV pole.

The processing unit 104 governs the interaction and relationship between the different subsystems to properly perform an IOL injection procedure. To do this, it includes a processor and memory and is preprogrammed with instructions for controlling the subsystems to carry out the IOL injection surgical procedure.

In one exemplary embodiment, the motorized handheld IOL injection device 112 is driven by the console 100 with surgeon control from the footpedal 108. It provides smooth and consistent injection control of the IOL. The display screen 106 accommodates setup and use of the handheld IOL injection device 112.

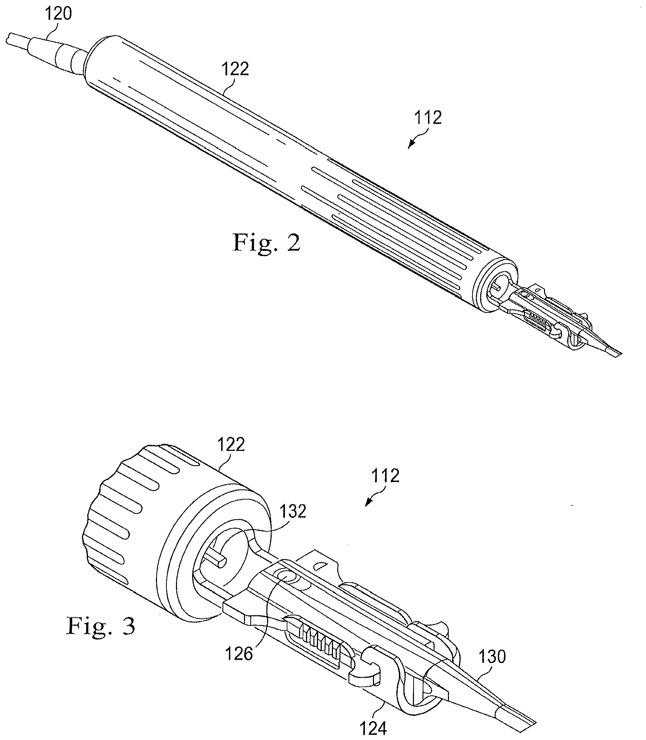

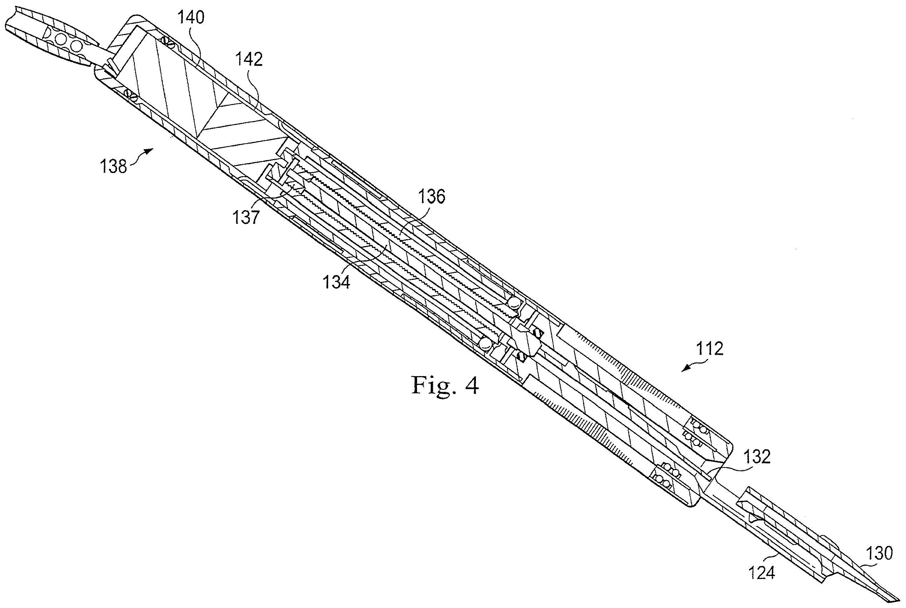

Figs. 2 and 3 illustrate the exemplary handheld IOL injection device 112 for implanting an IOL into the anterior capsule of the eye. Fig. 3 is merely a close-up of the distal end of the device shown in Fig. 2. As pictured, the IOL injection device 112 includes a cable assembly 120 that carries power and/or control signals from the console 100 and a main housing 122 that may serve as a hand grip to be grasped by a surgeon during use. The housing includes a distal or front end and proximal or back end. In the example shown, the cable assembly 120 is disposed at the back end. Instead of having the cable assembly 120, some embodiments may include one or more batteries in the main housing 122 to provide electrical power to the device and/or one or more switches or other user input devices to control the operation of the device. The exemplary IOL injection device 112 also comprises a cartridge mount 124 disposed at the distal or front end of the housing 122, which holds a removably mounted insertion cartridge 130. The insertion cartridge 130 in some embodiments is a disposable polymeric component adapted to accommodate an unfolded IOL lens 126 and to fold and displace the lens as a plunger tip 132 of a plunger (Fig. 4) is translated forward from the body of the housing 122 and through the insertion cartridge 130.

Figs. 4-6 illustrate cross-sectional views of the IOL injection device 112 according to some embodiments of the present invention. Referring first to Fig. 4, the IOL injection device 112 includes the plunger tip 132, a plunger 134, an internally threaded tubular coupler 136, a male coupler 137, and an electric drive system 138. The plunger 134 is configured to longitudinally translate inside the internally threaded tubular coupler 136 during actuation of the drive system 138. The electric drive system 138 may comprise an electric motor 140 and a gear set 142 disposed within a weldment and configured to rotate the tubular coupler 136. In the exemplary embodiment shown, internal threads on the tubular coupler 136 engage the externally threaded male coupler 137 at the rear end of the plunger 134, forcing linear translation of the plunger 134 and plunger tip 132 within the tubular coupler 136, in response to activation of the drive system 138. However, other electric drive systems for advancing the plunger 134 with an electric motor are contemplated.

In some embodiments, the electric drive system 138 comprises a brushless DC motor 140 that provides rotational torque to the gear set 142, which in turn rotates the tubular coupler 136 to extend or retract the plunger 134. The gear set 142 is effective to reduce the angular velocity of the motor according to a pre-determined reduction ratio, e.g., 125:1. This increases the available torque from the drive system 138, and slows the linear motion of the plunger 134 to a speed appropriate for the IOL injection procedure. Additional details relating to the operation of the IOL injection device 112 may be found in U.S. Patent Application No. 12/249,996 to Boukhny et al.

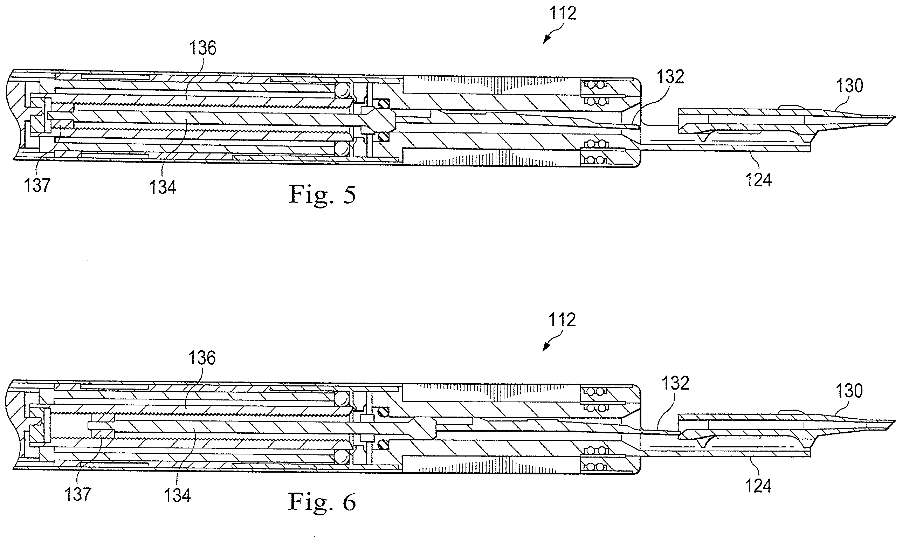

Figs. 5 and 6 illustrate a longitudinal cross-section of the IOL injection device 112 with the plunger 138 in a fully retracted position and in a partially extended position, respectively. In the partially extended position illustrated in Fig. 6, the plunger tip 132 is just beginning to pass into the insertion cartridge 130, where it will engage the IOL maintained within the insertion cartridge and will advance the IOL toward the distal end of the cartridge and ultimately into the patient.

The insertion cartridge 130 maintains the IOL 126 (Fig. 3) in a position ready for use. The insertion cartridge 130 is loaded onto the IOL injection device 112 in a manner that the plunger tip 132 can axially advance and engage the IOL in the proximal end of the cartridge 130. As the plunger 134 advances through the insertion cartridge 130, the plunger pushes the IOL through a tapering passage that deforms the IOL into an elongated condition with a narrow width. In this form the IOL may be pushed out of the end of the cartridge 130 and into the implantation site of the patient's eye, where it will re-expand to take on its substantially cylindrical shape.

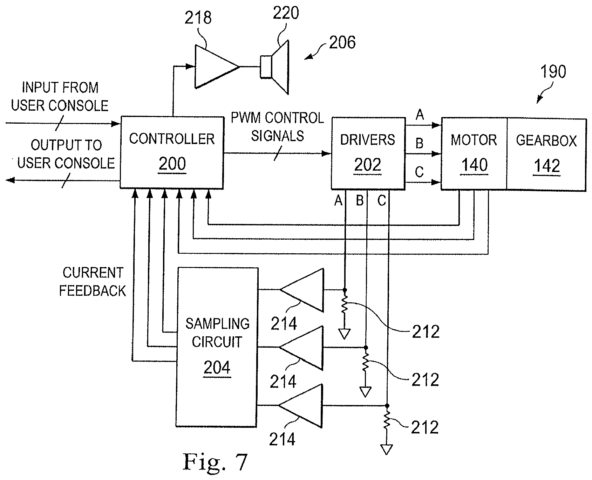

Fig. 7 discloses an exemplary control circuit 190 for implanting an IOL into an eye of the patient. The control circuit 190 monitors the electric motor current as an indication of the load on the motor 140 and may continuously provide feedback to the surgeon and/or modify or halt movement of the plunger 134 (Fig. 4) if an adverse condition is detected based on the load. The control circuit 190 detects and monitors the motor current feedback. The current feedback is directly proportional to torque on the motor, which is representative of load. Therefore, as the load on the motor increases, or as the force required to operate the motor increases, the electric current increases. Likewise, as the load on the motor decreases, or as the force required to operate the motor decreases, the electric current decreases. Because of this, monitoring the actual load on the motor can provide an indication of whether the insertion procedure is progressing according to expected norms.

The control circuit 190 includes a controller 200, drivers 202, a sampling circuit 204, and an audible indicator 206. The controller 200 may include a control processor portion and may include or be associated with a memory having stored therein one or more preferred motor-load profiles. A motor-load profile may be a profile indicating an expected load on the motor 140 during an optimal IOL insertion procedure. For example, due to the geometry of the intraocular lens and the volume of viscoelastic injected into the insertion cartridge, a properly loaded cartridge has a unique inherent viscous resistance to the plunger, and thus provides a known load on the motor 140. When compared to a loaded cartridge, the empty cartridge also has a distinct load signature. Because of the relationship between torque and load in a DC motor, an increase in the load is reflected in an increase in the torque and in a higher motor current, for a given drive level. Conversely, a decrease in the load is reflected in a decrease in torque and a decrease in motor current. Because the current drawn by the motor 140 is directly proportional to the motor's torque, the current level can be monitored to determine the torque, and hence the applied load.

In an optimal procedure, the actual load on the motor throughout an IOL insertion procedure mimics the expected load set out in the motor-load profile. Deviation of the actual load on the motor from the expected load may signify an adverse or fault condition, such as an occluded IOL cartridge or other condition that would cause an unexpected deviation in load.

In some embodiments, the expected load may be established by one or more thresholds or by an envelope about a particular expected load. These would provide a range for the expected load. As the actual load deviates from the expected load, the system may provide real-time feedback to the surgeon. Modification thresholds, such as stop thresholds, may be included that modify, stop, or prevent movement of the plunger when deviation of the actual loads from the expected loads exceeds the modification thresholds. For example, the modification thresholds may be pre-set offsets from the expected loads. In some embodiments, the expected loads and modification thresholds are pre-determined, e.g., by factory calibration, and stored in memory in or accessible to controller 200. (Those skilled in the art will appreciate that this memory may comprise program memory or a separate memory storing factory-determined parameters or the like, and may comprise any of several conventional memory types, including ROM (Read-Only Memory), PROM (Programmable Read-Only Memory), EEPROM (Electrically Erasable Programmable Read-Only Memory), flash, etc.)

In some embodiments, the modification thresholds comprise a plurality of thresholds. For example, when the actual load deviates from the expected load by a first amount, the controller 200 may decrease speed of the plunger. When the actual load deviates from the expected load by a second greater amount, the controller 200 may halt the plunger. When the actual load deviates back toward the expected load, the speed may increase to the original speed. Any number of thresholds may be stored for fine adjustment and control.

The controller 200 produces pulse-width modulated (PWM) control signals for commutating the motor 140, and the drivers 202 convert the digital control signals into analog drive signals applied to the stator winding inputs A, B, and C.

The sampling circuit 204 monitors electric current being sent to the motor's winding inputs A, B, and C. In some embodiments, the sampling circuit 204 includes analog-to-digital converters to convert motor current at the drivers 202 to digital signals for use by controller 200. In some embodiments, the sampling circuit 204 may be synchronized to the PWM control signals produced by controller 200. However, those skilled in the art will appreciate that in other embodiments the motor inputs may be sampled over the entire duty cycle, and the current signals may be isolated by digital processes in the controller 200.

The sampling circuit 204 connects to the drivers 202 via connections including resistors 212. Amplifiers or conditioning circuitry 214 treat the current for more accurate detection at the sampling circuit 204. The current feedback is then communicated to the controller 200.

In some embodiments, the controller 200, the drivers 202, and the sampling circuit 204 are maintained on the surgical console 100 (Fig. 1) and communication to the motor 140 occurs via the cable assembly 120 (Fig. 2). In other embodiments, the circuitry is maintained on the handheld IOL injection device itself, while in yet other embodiments, the circuitry is disposed elsewhere about the system. In some aspects, the sampling circuit 204 is a part of the controller 200. The controller 200 may form a part of or may be separate from the main processing unit 104 of the surgical console 100.

The audible indicator 206 may also be on the console 100, on the IOL injection device 112, or at another location where it may be heard by a surgeon. It is configured to provide a sensory signal to the surgeon by providing audible feedback relating to the detected load. In one embodiment, the audible feedback is a continuous tone that varies in pitch as the load changes. For example, when the load is at an initial value within an acceptable range, the audible feedback may produce a tone at a first pitch. As the load changes, the pitch may correspondingly change. An increasing or higher pitch may indicate an increasing or higher load, and a decreasing or lower pitch may indicate a decreasing or lower load. In some embodiments, the tone changes only as the actual load deviates from the expected load. Accordingly, in these embodiments, since the load is expected to change as the plunger engages the IOL, the controller 200 may control the audible indicator to have a relatively constant pitch. Likewise, as the actual load deviates from the expected load, the pitch may increase or decrease to alert the surgeon to the changing conditions.

In another aspect, instead of a continuous tone, the audible indicator 206 may emit an intermittent tone, where the frequency of the tones increases as load increases and decreases as the load decreases. Accordingly, a surgeon may receive real-time feedback regarding the current load level by how fast the tone is beeping.

The audible indicator 206 may comprise an amplifier 218 and a speaker 220. In addition to the audible indicator 206, some embodiments of the system may be designed to automatically cease advancement of the IOL when the load exceeds expected load values by more than an acceptable amount.

Alternative embodiments include non-audible indicators that alert the surgeon when conditions fall outside expected ranges or exceed the modification thresholds. Some indicators are visual indicators, such as an indicator light that may flash or a message on the display screen. Yet others provide tactile indicators, such as vibration at the foot-pedal. Still other indicators are also contemplated.

By comparing the data from the current level at a given instance to the predetermined threshold of the motor-load profile, the controller 200 can detect whether or not the motor is operating at its expected load. Thus, the controller 200 can detect faults in operation and automatically respond (e.g., by shutting down) and/or providing feedback to the user.

For example, a load cartridge containing less than the required viscoelastic in the cartridge will result in a low torque and a corresponding current level lower than an expected level, in which case the controller 200 can notify the user. Conversely, when the torque and corresponding current value is higher than an expected level, it suggests an occluded cartridge. Again, the operation of the device can be shut down, and appropriate notice provided to the user.

In some embodiments, the longitudinal position of the plunger 134 can be tracked. This permits the system to correlate the detected torque to different stages of the insertion process. For example, as the tip of the plunger 134 is approaching the cartridge, the plunger is expected to move with little resistance. Accordingly, the expected load will be low based on the low current drawn by the motor. Once the plunger engages the cartridge, the load, and therefore, the current, increases.

Further, as the IOL advances, and is compacted to cause elastic deformation in the IOL, the load would continue to increase. In one embodiment, the position of the plunger is measured directly using a linear or rotary encoder that provides information regarding the actual location of the plunger.

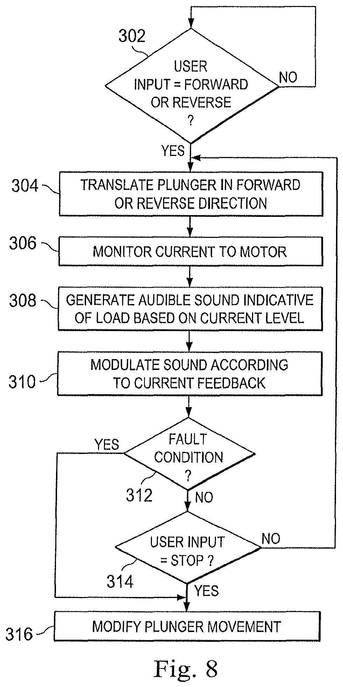

With the preceding discussions in mind, those skilled in the art will appreciate that the process flow diagram of Fig. 8 illustrates an exemplary method for controlling an intraocular lens injection device. Those skilled in the art will appreciate that this particular process flow is not intending to be limiting; numerous variations of this method falling within the scope of the present invention will be apparent in view of the preceding discussion. Those skilled in the art will further appreciate that the processing flow of FIG. 8 may be implemented in software or firmware stored in program memory within or associated with the controller 200 or the processing unit 104, for example, which memory may comprise one or more of various conventional types including read-only memory (ROM), programmable read-only memory (PROM), flash memory, magnetic or optical memory devices, or the like.

In any case, the process flow illustrated in Fig. 8 begins with the IOL injection device 112 in an inactive state. The device 112 checks for user input indicating that actuation of the plunger should begin, as shown at 302. This user input may originate at any of a number of conventional user input devices, such as a keypad or touchscreen at the surgical console 100, at the footpedal switch 108 electrically connected to the IOL injection device by cable or via a console, or one or more switches or buttons on the body of the IOL injection device itself. In any case, in response to user input indicating that the plunger assembly should be moved, the control circuit 190 begins translation of the plunger 134 in the indicated direction, as shown at 304.

As the plunger is moved, the current to the electric motor 140 is monitored, as shown at 306, according to any of the techniques discussed above. In some embodiments, the current level is monitored and compared to one or more pre-determined current thresholds, such as the expected loads and modification thresholds in the motor-load profiles discussed above.

At 308, the audible indicator 206 generates an audible sound that may be heard by the surgeon. The sound is indicative of the current level and is representative of load on the motor 140. In some embodiments, the sound is a continuous tone emitted to the surgeon while in other embodiments, the tone is an intermittent beep. Other audible indicators are also contemplated. In some embodiments, non-audible indicators, such as for example, alerts on the display and vibration through the foot-pedal are also contemplated.

At 310, the controller 200 modulates the sound generated at the audible indicator 206 according to the monitored current feedback. In so doing, the surgeon is kept informed of the relative loads on the plunger during the IOL insertion process. In one example, the tone is modulated based on the amount of deviation of the actual load from an expected load. Accordingly, the audible tone may be held consistent through the insertion process even through the actual loads may vary. For example, the loads on the plunger may be low while the plunger is advanced toward the IOL, and the expected load is likewise low. The load may increase when the plunger engages the IOL, and the expected load may likewise increase. Therefore, although the load changes, the deviation of the actual load from the expected load may be relatively constant. Accordingly, in the embodiment described, the tone may be maintained relatively constant so long as the actual load and the expected load change at the same relative rate. In other embodiments, the tone changes as the load changes regardless of the amount of deviation from the expected load.

Some embodiments use a continuous tone during the insertion procedure, while other embodiments employ an intermittent beeping sound. The frequency of the beeps or the beep rate may indicate the increasing or decreasing load. For example, as the load increases, the beep rate may also increase, and as the load decreases, the beep rate may also decrease. This beep rate may be based on deviation of the load from the expected load or simply on changes in the load.

If a fault condition is detected, as indicated at 312, the movement of the plunger is immediately suspended, as shown at 316. As discussed above, the detected fault condition may correspond to excessive resistance to forward or backwards movement of the plunger, compared to pre-determined threshold levels, or insufficient resistance to forward or backwards movement of the plunger, compared to pre-determined threshold levels. In any of these cases, the threshold level for fault detection may vary according to a tracked longitudinal position of the plunger, as indicated above. Furthermore, the operational threshold levels may be adjusted according to a baseline resistance or operating speed determined during a "no-load" condition.

In some embodiments, the plunger movement is decreased or increased based on the fault condition. Some embodiments have a plurality of stored modification thresholds, where detected current beyond intermediate modification thresholds results in a decrease of the speed of plunger movement and detected current beyond further modification thresholds results in stopped plunger movement. When the detected current returns from beyond the intermediate modification thresholds, the plunger speed may be increased to the original speed. The intermediate modification thresholds may have a value therefore, between the stop fault value and the operational threshold value.

In some embodiments, modification of the plunger's movement in response to a detected fault may be accompanied with or followed by an additional alert to the user, indicating the fault. For example, in some cases, a message identifying a particular type of fault (e.g., "blocked cartridge", "empty cartridge", or the like) may be provided to the user via the display 106 on the surgical console 100. If a fault condition is not detected at 312, then the status of the user input is checked, as shown at 314. If the user input indicates that movement of the plunger should be modified, then the motor may be slowed and the plunger's translation rate is correspondingly affected, as shown at 316. If the user input indicates that the modification should stop plunger movement, then the motor is deactivated and the plunger's translation is stopped. Otherwise, translation of the plunger continues, as shown at 314, and the preceding operations are repeated until either a fault occurs or the user input indicates that the plunger assembly's movement should be stopped.

In the above discussion of the process flow of Fig. 8, it was assumed that translation of the plunger continues, once initiated, until user input directs a stop or until a fault condition is detected. Those skilled in the art that the plunger motion may be limited at either or both ends by a mechanical stop. In some embodiments, these mechanical stops may be detected by the same fault detection mechanisms as described above, i.e., by monitoring the motor current levels. Alternatively, some embodiments may prevent the plunger from reaching the mechanical stops by tracking the longitudinal position of the plunger, as described above, and automatically stopping the plunger's movement before it reaches a mechanical stop.

As noted above, in some examples (not forming part of the invention), a method of controlling a system for implanting an intraocular lens in the lens capsule of an eye to treat an ocular condition may include (a) storing a motor load profile in a memory, (b) receiving an input signal to advance an intraocular lens disposed within an electrically-powered handheld IOL injection device having a motor configured to advance the intraocular lens, (c) advancing the intraocular lens, (d) detecting an actual load required to advance the lens by monitoring motor current feedback, (e) comparing the actual load to the stored motor load profile, and (d) modifying movement of the intraocular lens when the actual load deviates from the stored motor load profile by a pre-stored amount. In some embodiments, providing a sensory signal may include emitting an audible signal that changes in real-time to represent changes in the actual load. In some embodiments, the audible indicator may be a continuous tone. In some embodiments, the audible indicator may be an intermittent beeping sound. In some embodiments, providing a sensory signal may include emitting an audible indicator that changes in real-time to represent an amount of deviation from the actual load. In some embodiments, modifying movement of the intraocular lens may include stopping movement of the intraocular lens.

Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure

## Claims

1. A system (100) for implanting an intraocular lens in a lens capsule of an eye to treat an ocular condition, the system comprising:
a housing (122) having a primary axis extending between front and rear ends of the housing;
a plunger (134) longitudinally disposed within the housing and having first and second ends, the first end being disposed towards the front end of the housing;
an electric motor (140) configured to cause longitudinal translation of the plunger along the primary axis of the housing;
a cartridge mount (124) at or near the front end of the housing (122) and configured to accommodate a removable insertion cartridge (130) in alignment with the plunger so that the intraocular lens disposed in the insertion cartridge is displaced from the insertion cartridge as the plunger is translated towards the front end of the housing; and
a control circuit (190 comprising a controller (200) electrically communicating with the electric motor and configured to power the electric motor to translate the plunger and displace the intraocular lens, the controller configured to detect motor current feedback in the control circuit and configured to compare the detected motor current feedback to a stored current profile and to modify translation of the plunger when a current level deviates from the stored current profile by a pre-stored amount.

2. The system of claim 1, further comprising an audible indicator (206) configured to alert a user when the detected motor current feedback deviates from the stored current profile.

3. The system of claim 2, wherein the audible indicator (206) continuously generates sound at a variable pitch, the pitch varying in response to variances in the detected current feedback.

4. The system of claim 1, wherein the stored current profile represents an expected load on the motor when translating the plunger and displacing the intraocular lens.

5. The system of claim 1, further comprising a sensor disposed to detect a longitudinal position of the plunger.

6. The system of claim 1, wherein the controller comprises a memory portion storing the stored current profile, the stored current profile representing an expected load on the motor, wherein the controller is configured to compare an actual load based on the detected motor current feedback to the stored expected load.

7. The system of claim 1, wherein the stored current profile represents an acceptable range having an upper limit and a lower limit.

8. The system of claim 1 configured to compare the detected motor current feedback to a stored current profile and to emit a sensory signal to a surgeon indicative of the detected current feedback.

9. The system of claim 8, wherein the control circuit is configured to modify translation of the plunger when a current level deviates from the stored current profile by a pre-stored amount.

10. The system of claim 8, wherein the sensory signal is an audible indicator that continuously generates sound at a variable pitch, the pitch varying in response to variances in the detected current feedback.

11. The system of claim 3 or 10, wherein the pitch is configured to vary based on changes in a motor load based on the detected motor feedback.

12. The system of claim 3 or 10, wherein the pitch is configured to vary based on deviation of an actual load based on the detected motor feedback from the stored current profile.

13. The system of claim 2 or 10, wherein the audible indicator generates a sound at a variable rate, the rate varying in response to variances in the detected motor current feedback.

14. The system of claim 2 or 10, wherein the audible indicator is configured to provide real-time feedback regarding changes in load to the surgeon.

## Patentansprüche

1. System (100) zum Implantieren einer Intraokularlinse in eine Linsenkapsel eines Auges zur Behandlung einer Augenkrankheit, wobei das System umfasst:
ein Gehäuse (122) mit einer Primärachse, die sich zwischen dem vorderen und dem hinteren Ende des Gehäuses erstreckt;
einen Kolben (134), der in Längsrichtung innerhalb des Gehäuses angeordnet ist und erste und zweite Enden aufweist, wobei das erste Ende in Richtung des vorderen Endes des Gehäuses angeordnet ist;
einen Elektromotor (140), der ausgestaltet ist, um Translation des Kolbens in Längsrichtung entlang der Primärachse des Gehäuses zu bewirken;
eine Kartuschenhalterung (124) an oder nahe dem vorderen Ende des Gehäuses (122), und die ausgestaltet ist, um eine herausnehmbare Einführkartusche (130) ausgerichtet mit dem Kolben aufzunehmen, so dass die in der Einführkartusche angeordnete Intraokularlinse aus der Einführkartusche heraus verschoben wird, wenn der Kolben in Richtung des vorderen Endes des Gehäuses translatiert wird; und
eine Steuerschaltung (190), die eine Steuerung (200) umfasst, die elektrisch mit dem Elektromotor kommuniziert und ausgestaltet ist, um den Elektromotor mit Energie zu versorgen, um den Kolben zu translatieren und die Intraokularlinse zu verschieben, wobei die Steuerung ausgestaltet ist, um in der Steuerschaltung Rückmeldung des Motorstroms zu detektieren, und ausgestaltet ist, um die detektierte Rückmeldung des Motorstroms mit einem gespeicherten Stromprofil zu vergleichen und die Translation des Kolbens zu modifizieren, wenn ein Stromniveau um einen vorab gespeicherten Betrag von dem gespeicherten Stromprofil abweicht.

2. System nach Anspruch 1, des Weiteren umfassend einen hörbaren Indikator (206), der ausgestaltet ist, um einen Anwender zu alarmieren, wenn die detektierte Rückmeldung des Motorstroms von dem gespeicherten Stromprofil abweicht.

3. System nach Anspruch 2, wobei der hörbare Indikator (206) kontinuierlich Schall mit einer variablen Tonhöhe generiert, wobei die Tonhöhe in Reaktion auf Varianzen in der detektierten Rückmeldung des Stroms variiert.

4. System nach Anspruch 1, wobei das gespeicherte Stromprofil eine erwartete Motorlast repräsentiert, wenn der Kolben translatiert wird und die Intraokularlinse verschoben wird.

5. System nach Anspruch 1, des Weiteren umfassend einen Sensor, der angeordnet ist, um eine längsgerichteten Position des Kolbens zu detektieren.

6. System nach Anspruch 1, wobei die Steuerung einen Speicheranteil umfasst, welcher das gespeicherte Stromprofil speichert, wobei das gespeicherte Stromprofil eine erwartete Motorlast repräsentiert, wobei die Steuerung ausgestaltet ist, um eine tatsächliche Last, die auf der detektierten Rückmeldung des Motorstroms basiert, mit der gespeicherten erwarteten Last zu vergleichen.

7. System nach Anspruch 1, wobei das gespeicherte Stromprofil einen annehmbaren Bereich mit einem oberen Grenzwert und einem unteren Grenzwert repräsentiert.

8. System nach Anspruch 1, das ausgestaltet ist, um die detektierte Rückmeldung des Motorstroms mit einem gespeicherten Stromprofil zu vergleichen und ein sensorisches Signal an einen Chirurgen auszugeben, welches die detektierte Rückmeldung des Stroms angibt.

9. System nach Anspruch 8, wobei die Steuerschaltung ausgestaltet ist, um die Translation des Kolbens zu modifizieren, wenn ein Stromniveau um einen vorab gespeicherten Betrag von dem gespeicherten Stromprofil abweicht.

10. System nach Anspruch 8, wobei das sensorische Signal ein hörbarer Indikator ist, der kontinuierlich Schall mit einer variablen Tonhöhe generiert, wobei die Tonhöhe in Reaktion auf Varianzen in der detektierten Rückmeldung des Stroms variiert.

11. System nach Anspruch 3 oder 10, wobei die Tonhöhe so ausgestaltet ist, dass sie basierend auf Änderungen in einer Motorlast variiert, die auf der detektierten Rückmeldung des Motors basiert.

12. System nach Anspruch 3 oder 10, wobei die Tonhöhe so ausgestaltet ist, dass sie basierend auf Abweichung einer tatsächlichen Last, die auf der detektierten Rückmeldung des Motors basiert, von dem gespeicherten Stromprofil basiert.

13. System nach Anspruch 2 oder 10, wobei der hörbare Indikator einen Schall mit einer variablen Rate generiert, wobei die Rate in Reaktion auf Varianzen in der detektierten Rückmeldung des Motorstroms variiert.

14. System nach Anspruch 2 oder 10, wobei der hörbare Indikator ausgestaltet ist, um dem Chirurgen Rückmeldung in Echtzeit hinsichtlich Änderungen in der Last bereitzustellen.

## Revendications

1. Système (100) pour l'implantation d'une lentille intraoculaire dans une capsule du cristallin d'un œil pour traiter une pathologie oculaire, le système comprenant :
un boîtier (122) possédant un axe principal s'étendant entre des extrémités avant et arrière du boîtier ;
un piston (134) disposé longitudinalement à l'intérieur du boîtier et possédant des première et seconde extrémités, la première extrémité étant disposée en direction de l'extrémité avant du boîtier ;
un moteur électrique (140) conçu pour provoquer une translation longitudinale du piston le long de l'axe principal du boîtier ;
une monture (124) de cartouche au niveau ou à proximité de l'extrémité avant du boîtier (122) et conçue pour accueillir une cartouche d'insertion amovible (130) alignée sur le piston de sorte que la lentille intraoculaire disposée dans la cartouche d'insertion soit déplacée depuis la cartouche d'insertion à mesure que le piston est déplacé par translation en direction de l'extrémité avant du boîtier ; et
un circuit de commande (190) comprenant un organe de commande (200) communiquant électriquement avec le moteur électrique et conçu pour alimenter le moteur électrique afin de déplacer par translation le piston et de déplacer la lentille intraoculaire, l'organe de commande étant configuré pour détecter une rétroaction de courant de moteur dans le circuit de commande et conçu pour comparer la rétroaction de courant de moteur détectée avec un profil de courant mémorisé et pour modifier une translation du piston lorsqu'un niveau de courant dévie du profil de courant mémorisé à hauteur d'une quantité préalablement mémorisée.

2. Système selon la revendication 1, comprenant en outre un indicateur sonore (206) configuré pour alerter un utilisateur lorsque la rétroaction de courant de moteur détectée dévie du profil de courant mémorisé.

3. Système selon la revendication 2, dans lequel l'indicateur sonore (206) génère en continu un son à un pas variable, le pas variant en réponse à des variances dans la rétroaction de courant détectée.

4. Système selon la revendication 1, dans lequel le profil de courant mémorisé représente une charge attendue sur le moteur lors de la translation du piston et du déplacement de la lentille intraoculaire.

5. Système selon la revendication 1, comportant en outre un capteur disposé pour détecter une position longitudinale du piston.

6. Système selon la revendication 1, dans lequel l'organe de commande comprend une partie mémoire mémorisant le profil de courant mémorisé, le profil de courant mémorisé représentant une charge attendue sur le moteur, dans lequel l'organe de commande est configuré pour comparer une charge réelle sur la base de la rétroaction de courant de moteur détectée avec la charge attendue mémorisée.

7. Système selon la revendication 1, dans lequel le profil de courant mémorisé représente une plage acceptable ayant une limite supérieure et une limite inférieure.

8. Système selon la revendication 1, conçu pour comparer la rétroaction de courant de moteur détectée avec un profil de courant mémorisé et pour émettre vers un chirurgien un signal sensoriel faisant état de la rétroaction de courant détectée.

9. Système selon la revendication 8, dans lequel le circuit de commande est configuré pour modifier une translation du piston lorsqu'un niveau de courant dévie du profil de courant mémorisé à hauteur d'une quantité préalablement mémorisée.

10. Système selon la revendication 8, dans lequel le signal sensoriel est un indicateur sonore qui génère en continu un son à un pas variable, le pas variant en réponse à des variances dans la rétroaction de courant détectée.

11. Système selon la revendication 3 ou 10, dans lequel le pas est configuré pour varier sur la base de changements d'une charge de moteur sur la base de la rétroaction de moteur détectée.

12. Système selon la revendication 3 ou 10, dans lequel le pas est configuré pour varier sur la base d'un écart d'une charge réelle sur la base de la rétroaction de moteur détectée à partir du profil de courant mémorisé.

13. Système selon la revendication 2 ou 10, dans lequel l'indicateur sonore génère un son à une fréquence variable, la fréquence variant en réponse à des variances dans la rétroaction de courant de moteur détectée.

14. Système selon la revendication 2 ou 10, dans lequel l'indicateur sonore est configuré pour fournir au chirurgien une rétroaction en temps réel concernant des changements de charge.
